# EUROPEAN PATENT APPLICATION

(11) **EP 2 727 536 A1**
(43) Date of publication of application: **07.05.2014**
(21) Application number: 13190948.3
(22) Date of filing: 30.10.2013
(51) Int. Cl.: A61B 6/04, A61B 6/00

(54) **Device for X-ray examinations**

(30) Priority: 31.10.2012 SE 1251221
(71) Applicant: Medical Innovation Design MID AB, 135 54 Tyresö (SE)
(72) Inventor: Ryngebo, Ylva, 135 54 Tyresö (SE)
(74) Representative: Höglund, Lars

(57) **Abstract**

A head and arm support, a system and/or a device that is transmissive for X-ray radiation having a space for receiving a detector, for use in radiographic examinations of patients who cannot help themselves to position itself due to sickness or unconsciousness or immobility. The head and arm support, system or device facilitates the work for the hospital staff and also the comfort for the patient. The device and the head and arm support are also releasably connectable to each other and can therefore be used separately.

## Description

### Technical field

The present invention relates to the field of devices for use in X-ray examinations.

### Background art

When a patient undergoes X-ray examination of the chest or lung area, x-ray images must be taken from a side view, and from the front and/or back. The patient must also hold his arms so they do not obscure the x-ray image. This is not a problem as long as the patient is able to help along and cooperate with the staff. However, when a patient is very sick or unconscious the patient is not able to help along, cooperate or stand up for performing a proper X-ray. The patient is then lying on a bed or the like and the hospital staff must help the patient to be in the correct position and also put the cassette or detector underneath the patient. The arms of the patient must also in some way be hold in a position so that they do not obscure the x-ray image during the exposure.

Today, there are no useful means that helps the hospital staff to facilitate X-ray examinations of the chest and lung area on very sick or unconscious patients. Known devices are quite heavy, non-hygienic and/or difficult to use for the hospital staff. The hospital staff(s) must often also stay in the examination room holding the patients' arms in the right position during the x-ray exposure, sometimes the arms are taped in the right position, e.g., over the head or in a position not obscuring the chest area of the patient. Moreover, there is often necessary to repeat the x-ray examination until a sufficiently good X-ray image is obtained leading to unnecessary exposure of radiation to both the hospital staff as well as the patients'. Especially the life dose of radiation for the hospital staff is necessary to decrease due to for example higher cancer risk for people working with radiation or in a radiation environment.

To move, lift and hold a patient that is not able to help along due to sickness or unconsciousness is very heavy for the hospital staff. The heavy work also causes injuries to the hospital staff, which is costly for society in view of both sick leaves and rehabilitation cost. In addition, the very sick or unconscious patient is very heavy and the body tonus is also very low which makes the patient more heavy and difficult to get in the right position, and therefore there is also a higher risk that the patient is injured or even may fall down on the floor.

There are many steps during X-ray examinations that are in need of being improved, both in view of the hospital staffs' situation as well as in view of the patients undergoing the examination. Improved means would be important both for reducing sick-leaves and rehabilitation caused by heavy work but also reduce hospitalization cost of patients due to more effective and efficient appliances.

In view of the above there are indeed needs to find new means or improve means used today in the health care to decrease the risk for injuries related to heavy work of the hospital staff. Moreover, unnecessary radiation of the staff (as well as of the patients) must be avoided. One way to solve these problems is to improve the means used by the staff thereby facilitating their work, minimize their exposure to radiation as well as increasing the safety, comfort and respect for the patients.

### Summary of invention

A general aspect of the invention is to provide a head and arm support for a patient that is undergoing an X-ray examination, said support can also have attached releasable holding means for holding the patients arms in the correct position for a chest or lung X-ray, i.e., over the head.

Other uses of the support in medical applications than X-ray examinations are also possible.

In one embodiment the head and arm support comprises a top plate, a base plate, and fastening means releasably connecting the top and base plate to each other.

In another embodiment the head and arm support comprises at least one opening for receiving holding means.

In yet another embodiment the head and arm support comprises at least one holding means for holding for example the arms of a patient.

In one embodiment the at least one holding means is releasably attached to the top plate.

In yet another embodiment the at least one holding means is adjustable.

In yet another embodiment the at least one holding means is a strap.

In another embodiment the at least one holding means comprises a wrist girdle.

In another embodiment the at least one wrist girdle is designed so that a catheter or any hose or cord fits comfortably and securely under the wrist girdle.

In one embodiment the top plate is bent but in others embodiments the top plate is made of a top portion and a front portion.

In one embodiment the head and arm support is made of a disinfectable material, but other material may also be used.

In yet another embodiment the head and arm support comprises a mark, marking a predetermined side of the patient, the mark is also visible in the resulting image.

In another embodiment is all the material used made of disinfectable material.

A second object of the present invention is to provide a system for facilitating the X-ray examination procedure of the chest and lung area of sick, unconscious, paralyzed and/or immobile patients.

One embodiment of the system for use in radiographic examinations comprises a device that is transmissive for X-ray radiation, and a head and arm support as described above. The device comprises a space for releasably receiving a detector. The device and the head and arm support are also releasably connectable to each other.

In yet another embodiment of the system a mark is attached to the device for marking for example the left side of the patient.

The head and arm support of the system comprises a top plate, a base plate, and fastening means releasably connecting the top and base plates to each other, and in one embodiment the head and arm support is made of a disinfectable material.

In one embodiment is the device of the system designed to be capable of having a mattress on the top.

In one embodiment of the system the head and arm support is made of PVC.

In another embodiment of the system the head and arm support comprises at least one holding means releasably attached to the top plate.

In one embodiment of the system the head and arm support comprises at least one holding means in the form of a strap.

In yet another embodiment of the system the head and arm support comprises at least one holding means that is adjustable.

In another embodiment of the system the head and arm support comprises at least one holding means comprising at least one wrist girdle.

In yet another embodiment of the system the at least one holding means with the at least one wrist gridles of the head and arm support comprise a fastening means.

In one embodiment the fastening means (37) is designed as a bracket.

In one embodiment of the system the top plate of the head and arm support is bent or made of a top portion and a front portion.

A third object of the invention is to provide a device that is transmissive for X-ray radiation for use in radiographic examinations comprising:
- a first plate forming the bottom plate of the device;
- a second plate forming a wall in the head end of the device, and the device is designed to receive a releasable X-ray film, cassette, plate or detector wherein the head end is higher than the other end of the device.

In one embodiment the device has a weight less than about 7.5 kg, more preferably less than about 5 kg, and even more preferably less than about 3 kg.

In another embodiment comprise the second plate at least one opening suitable for receiving fastening and/or connecting means and/or for engagement of a mattress or another device.

In another embodiment the device is releasably connectable to other means, preferably a head and arm support.

In yet another embodiment the device has a premarked left and/or right side.

In one embodiment the system is i.e., the device and the head and arm support, also adapted to receive a mattress and/or pillow (that may be made in a disinfectable and/or durable material).

In one embodiment of the system the device comprises a first plate forming a bottom plate of the device, a second plate forming a wall in an upper end of the device facing the head and arm support, a third plate forming a top plate, and a fourth plate forming a wall in a lower end of the device, opposite to the upper end, wherein the height of the second plate is higher than the height of the fourth plate so that the upper end is higher than the lower end of the device.

In yet another embodiment at least one of the plates of the device comprises at least one opening for receiving fastening and/or connecting means for engagement of a mattress or another device.

In one embodiment of the device at least one of the plates is made of a composite material.

In yet another embodiment of the device in the system at least one of the plates is made of a PVC material.

Another object of the invention is to provide a device that is to be positioned underneath the patient during radiographic examinations. The device is transmissive for X-ray radiation and designed to hold a cassette or detector during X-ray examinations. The cassette or detector is also exchangeable to another one if there is a need for repeating the X-ray examination without need to lift or move the patient for exchanging the cassette or detector.

In another embodiment of the device at least one of the plates is made of a composite material.

In yet another embodiment of the device at least one of the plates is made of a PVC material.

In further another embodiment the top plate of the device is made of a composite material.

In one embodiment the space of the device is designed to receive a releasably X-ray film, cassette, plate or detector, but the space may also be designed in a different way and also be used for other purposes.

In one embodiment of the device the weight is less than about about 7.5 kg, and yet another embodiment less than about 5 kg.

In another embodiment of the device the weight is less than about less than about 3 kg.

In one embodiment the device the second side wall of the device comprise at least one opening for releasably receiving fastening means.

In yet another embodiment the device comprise several openings for receiving another device, fastening means and/or holding means.

In one embodiment the device is preferably made of a disinfectable material, but other suitable material can also be used.

In one embodiment the device may also be releasably connectable to other means.

In another embodiment the device is designed to be capable of receiving a mattress on the top side, but other things may also be put on the top such as for example a protection or a blanket.

In one embodiment of the device the releasably connectable means is a head and arm support.

In yet another embodiment the device has a mark is attached to the device for marking for example the left side of the patient, the mark is visible on the resulting image of the X-ray examination.

In view of the present application the word X-ray examination means a procedure wherein a patient is undergoing an X-ray examination of the chest and/or lung area by any available X-ray methods.

The word X-ray image is used for any kind of image that is obtained when using any X-ray exposure method, i.e., from old fashion X-ray detectors to digital equipment such as digital radiography.

The detection of X-rays is based on various methods, and regardless of what is "catching" the image, they are all categorized as "Image Receptors" (IR). The word detector is used for any image receptor such as for example photographic plates, photographic film, rare earth screens, flat panel detectors, flat panel displays, silicon drift detectors, medipix detectors, semiconductor detectors, solid state detectors, detectors.

The word cassette is used for any frame, holder or envelope or the like which has the purpose to hold the film or detector in place during the X-ray exposure. Said cassette is releasable from a space in the device. Some detectors do not need a cassette and can be put into the device directly.

The word fastening means is used for any means that may be used to connect one device to another, e.g., a mattress to a device or connecting a support to a device. The fastening means may be anything that connects two things or more to each other such as for example: hooks, washers, eyelets, buttons, snaps, Velcro, adhesive, zipper, magnetic, buckles.

The word holding means is used for any means that may be used to hold something in a certain position, such as holding the arms over the head of the patient. The holding means may be of any design suitable for holding the arms in the right position. The holding means may be of any suitable material, such as for example nylon, Velcro, vinyl, fabric, plastic foil.

The present invention provides a solution that counteracts some of the problems in prior art and thereby facilitating taking chest and/or lung x-rays on sick and/or unconscious patients.

In addition, all material used in the system, device or support can withstand disinfection. The material is durable and easy to wash. There are no protruding fastenings means that can collect dirt, or damage hospital staff or patients.

### Brief description of drawings

The invention is now described, by way of example, with reference to the accompanying drawings, in which:

Fig. 1A shows a perspective view of a head and arm support, 1B shows a side view of a fastener means. Fig. 1C shows a front view of a head and arm support, and fig.1D shows a perspective view of a head and arm support comprising arm holding means.

Fig. 2A shows a perspective view of a device transmissive for X-ray radiation, and fig. 2B show a side view of the same device.

Fig. 3A shows a perspective view a system comprising a head and arm support connected to a device that is transmissive for X-ray radiation. Fig 3B shows a side view of the same system as in fig. 3A. Fig. 3C shows a perspective view of a system comprising a cassette without a patient and fig. 3D shows the same system with a patient.

### Description of embodiments

Before the invention is disclosed and described in detail, it is to be understood that this invention is not limited to particular materials or configurations disclosed herein as such configurations and materials may vary. It is also to be understood that the terminology employed herein is used for the purpose of describing particular embodiments only and is not intended to be limiting since the scope of the present invention is limited only by the appended claims.

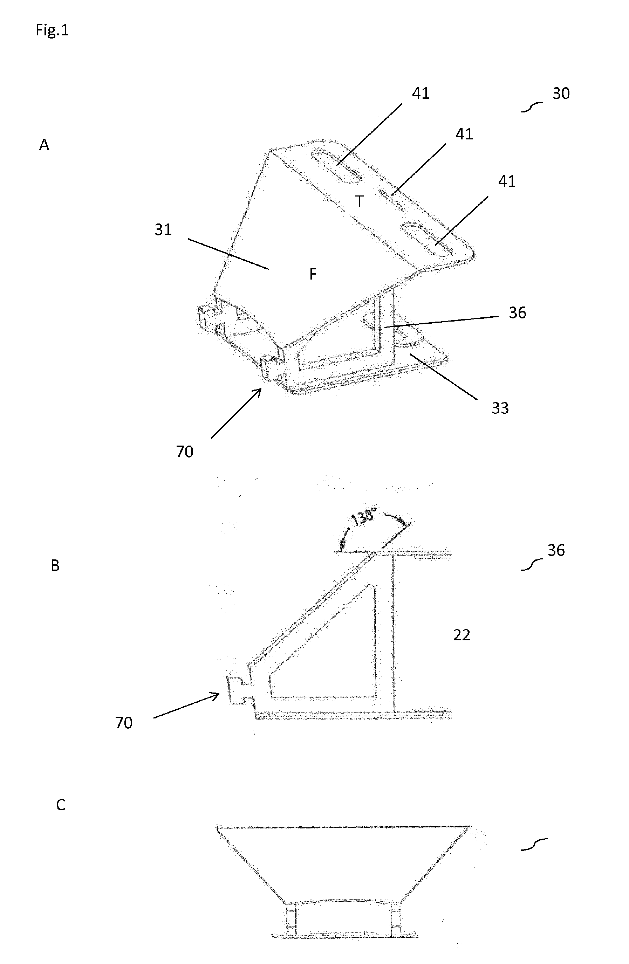

Figure 1A shows a perspective view of a head and arm support 30. The head and arm support 30 comprises a top plate 31, and a base plate 33. The top plate 31 and base plate 33 are connected to each other via fastening means 36. The top plate 31 is somewhat bent for creating a smooth rounding without sharp edges, dividing the plate into a front part F and top part T. Other embodiments can have separated top T - and front F plates connected to each other or made in a single piece. The fastening means 36 connects and supports the top 31 and base 33 plates but is in this embodiment also used to connect the head and arm support 30 to another device. The top plate 31 and bottom plate 33 are in this embodiment made of PVC-plastic. But other suitable materials can also be used.

In other embodiments may also the top - and bottom plate be made in one piece.

The top plate T of plate 31 comprises at least one opening 41 for receiving holding means for example.

Fig. 1B shows a side view of a fastening means 36, one side 22 is higher than the other side and another device may be releasably connected to the head and arm support via the connection means 70. The connection means 70 may be positioned in any suitable position on the fastening means 36, and could also be embodied as openings or other types of connection means.

Fig. 1C shows a front view of the head and arm support 30.

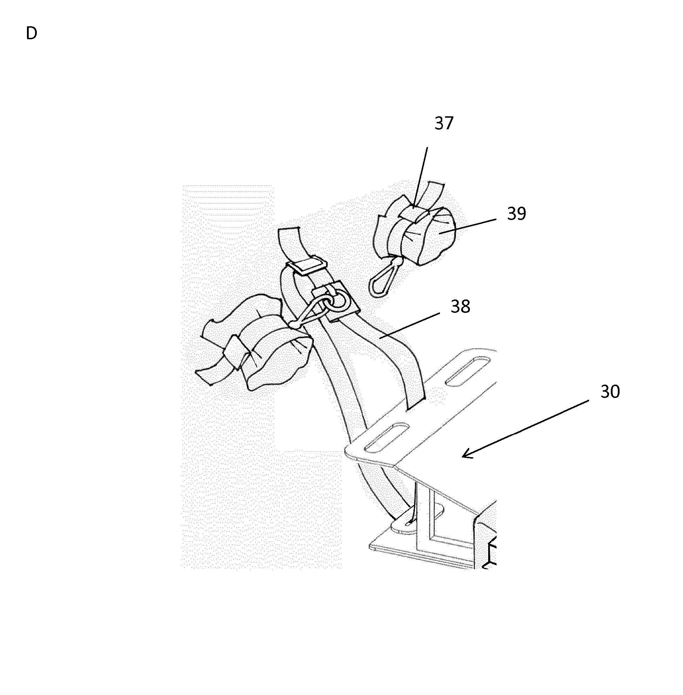

Fig 1D shows a perspective view of the head and arm support 30 with attached holding means 38 for holding the arms in the right position of a patient. The holding means 38 are in this embodiment made of an adjustable strap, but other embodiments such as for example using two straps are possible. An adjustable wrist holder is slidably attached to said strap. The wrist holder also comprises wrist girdles 39, one for respective arm, buckles 37 to fix the loops tight around the patients' wrists. The holding means 38 are releasably attached to the head and arm support 30. When the arms lies in the right position the length and position of the stripes are fixed by using fastening means such as for example a bracket, clips, magnetic, strap mount, buttons, Velcro or the like.

The holding means (38) are designed to be suitable for that a catheter or any hose or cord fits comfortably and securely under the wrist girdle (39).

All material used are durable and also possible to clean and disinfect which is very economic.

Moreover, there is no fastening means that protrude and can collect dirt that can cause serious infections in the hospital staff or patients.

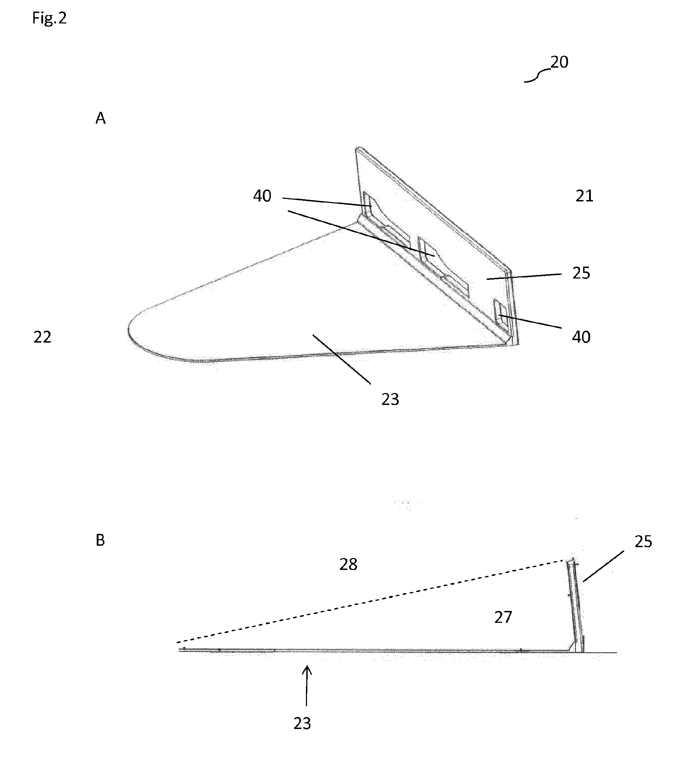

Fig. 2A shows a perspective view of a device 20 that is made of a material that is transmissive for X-ray radiation. The material in this embodiment is carbon composite material and plastics (PVC). It is possible to cast the device in whole or cast plate by plate in a casting mold or fix the plates to each other by using adhesive, bolts, pins, rivets or other commonly used fastening means. Due to the choice of material, the device can withstand heavy weights, such as a patient. The device is also remarkable light having a weight of less than 3 kg.

The device 20 comprises:
a first plate 23 forming the bottom plate of the device 20
a second plate 25 forming the wall in the upper end 21 of the device 20, and
a space 27 (or opening) for receiving and releasably holding a cassette comprising for example an X-ray film, or any detector. The plate 25 also comprise different premade openings 40 that are suitable as connection points for receiving fastening means from another device. The received device may be for example a head and arm support. The connection points can also receive fastening - or connection means 36, 37 from a mattress that is positioned on the top of the device for convenience.

The device 20 comprises an upper 21 and a lower 22 end, wherein the upper end 21 is higher than the lower end 22 of the device 20 thereby building up a slope 28 (Fig. 2B) that improves the position of the patient for X-ray examination of the chest and lung-area. The upper end 21 is facing the head end of the patient, and the lower end is facing the foot end of the patient. However the lower end is situated somewhere around the lower back area of the patient, depending on the length of the patient. The slope 28 is important for getting a good image from the side of the patient, but also important because it settles fluid that might be present in the lung in the lower part, thereby not obscuring the lung or chest area in the resulting image.

By adding a mattress, pillow, another plate or similar on the device a space 27 is created. The space 27 in the device is adapted to receive a cassette or detector. By using the device 20 it is possible to take a lot of images without having to lift and reposition the patient each time a new image must be taken.

Moreover, the device may also comprise a top plate made of carbon fiber material (composite material) the bottom plate 23 is made of a slidable material such as PVC plastic. It is important that the bottom material is slidably, since it should slide on the bed in under the patient. The front of the lower end 22 can be pointy which facilitates this movement. So, the design and choice of material of the device further facilitates for the hospital staff to put the device 20 in under the patient.

Optionally a mattress may be releasably connected to the device 20 by using fastening or connection means The fastening means may be buckles, but other types may be used such as for example bricks, buttons, flaps, snaps, straps, Velcro, magnetic or any adhesive. The material of the mattress may be PVC-coated fabric, the straps are made of nylon and the buckles are made of PVC, but other suitable materials can also be used. However, the material should be possible to disinfect and be durable against body liquids, liquids etc.

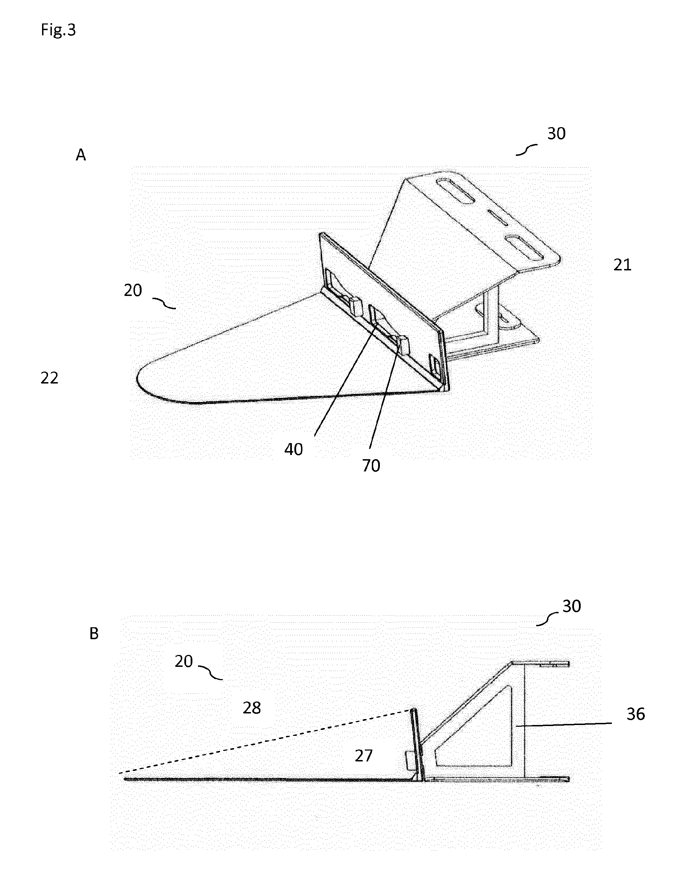

Fig. 3A-D shows a system to be used during X-ray examinations of a patient. The system comprises a device 20, and a head and arm support 30. The device 20, and the head and arm support 30 are releasably connectable to each other via connection points or openings 40, 70. The device 20 comprises an upper 21 and a lower 22 end, wherein the upper end 21 is higher than the lower end 22 of the device 20, thereby building up a slope 28 (Fig. 3B) that improves the position of the patient for X-ray imaging of the chest and lung-area. The space 27 in the device is adapted to receive a cassette or detector. A plate, pillow or mattress can be positioned over the space 27 making the device more comfortable to lie on.

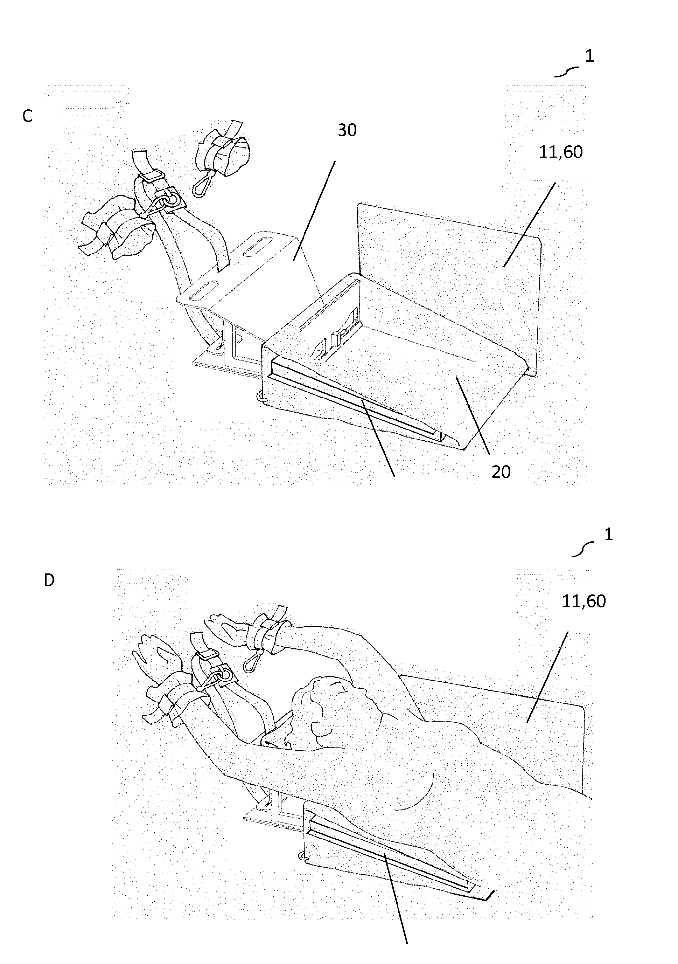

Figure 3C and 3D shows the system 1 in use without a patient (3C) and with a patient (3D). A detector 11, 60 may be situated on one side of the patient or inserted in the device 20. The space 27 formed when a mattress, pillow and/or a plate is on top of the device facilitates significantly exchange of a film or detector.

It is very important to mark a predetermined side of the patient when he or she is going to be radiographed. The mark shows where for example the left side of the patient is on the resulting image. This is very important to avoid mistakes where the left and right side of the patient is mixed. The left side (heart side) is usually marked in the case of chest and lung X-ray. Therefore it is also possible to attach a left-side mark permanently to the left side of the head and arm support (30) and/or the device (20). The mark(s) is also disinfectable.

The advantage of having a permanently attached mark is that the left and the right side of the patient cannot be mixed. Moreover, since the mark is attached to the device it will not disappear saving time since no one has to look for a mark.

Other features and uses of the invention and their associated advantages will be evident to a person skilled in the art upon reading the description and the examples.

## Claims

1. A head and arm support (30) for use in radiographic examinations **characterized in that** the head and arm support comprise:
- a top plate (31);
- a base plate (33); and
fastening means (36) releasably connecting the top and base plate to each other.

2. The head and arm support (30) according to claim 1, comprising at least one opening (41) to be able to bring at least one strap or any other fastening or holding means (38) through said openings.

3. The head and arm support (30) according to any of claim 1 or 2, comprising at least one holding means (38) for holding the arms of a patient, wherein the at least one holding means (38) may be releasably attached to the top plate, and wherein the at least one holding means (38) may be adjustable.

4. The head and arm support (30) according to any of claims 2-3, wherein the at least one holding means (38) comprises at least one wrist girdle (39).

5. The head and arm support (30) according claim 4, wherein the least one wrist girdle (39), comprises fastening means (37), preferably designed as a bracket.

6. The head and arm support (30) according to any of the preceding claims, wherein the head and arm support (30) is made of a disinfectable material.

7. The head and arm support (30) according to any claims 2-6, wherein the holding means (38) and the at least one wrist girdle (39) is made of a disinfectable material.

8. The head and arm support (30) according to any of claims 5-7, wherein the fastening means (37) is made of a disinfectable material.

9. The head and arm support (30) according to any of the preceding claims, having premarked left and/or right side.

10. A system (1) for use in radiographic examinations comprising:
- a head and arm support (30) according to any of claims 1-9,
- a device (20) that is transmissive for X-ray radiation, and
**characterized in that** the device (20) comprises a space (27) for releasably receiving a detector (11), and **in that** the device (20) and the head and arm support (30) are releasably connectable to each other.

11. A device (20) that is transmissive for X-ray radiation for use in radiographic examinations comprising:
- a first plate (23) forming the bottom plate of the device;
- a second plate (25) forming a wall in the head end of the device; and
- a space (27) of the device is designed to receive a releasable X-ray film, cassette, plate or detector (60),
wherein the head end (21) is higher than the other end (22) of the device.

12. The device (20) according to claim 11, wherein the weight of the device is less than about 7.5 kg, more preferably less than about 5 kg, and even more preferably less than about 3 kg.

13. The device (20) according to any of claims 11-12, wherein the second plate (25) comprise at least one opening (40) suitable for receiving fastening and/or connecting means (36 and/or 37) and/or for engagement of a mattress or another device.

14. The device (20) according to any of claims 11-13 that is releasably connectable to other means, preferably a head and arm support (30).

15. The device (20) according to claims 11-14, having premarked left and/or right side.
